# EUROPEAN PATENT APPLICATION

(11) **EP 1 156 063 A1**
(43) Date of publication of application: **21.11.2001**
(21) Application number: 01118103.9
(22) Date of filing: 08.02.1995
(51) Int. Cl.: C07K 16/28, C12N 5/20, A61K 39/395, A61P 35/00

(54) **Antibodies to the granulocyte colony stimulating factor receptor**

(30) Priority: 08.02.1994 AU PM375194
(62) Divisional of application: 95911668.2
(71) Applicant: LUDWIG INSTITUTE FOR CANCER RESEARCH, New York, New York 10158 (US)
(72) Inventor: Layton, Judith Eleanor, Clifton Hill, Victoria 3068 (AU)
(74) Representative: Harrison Goddard Foote

(57) **Abstract**

The present invention relates to cytokine receptor interactive molecules, their method of production and their use in the development of new therapeutic and diagnostic molecules. More particularly, the invention provides immunoreactive molecules such as antibodies wherein the antibodies are capable of interacting with granulocyte colony stimulating factor receptor.

## Description

### FIELD OF THE INVENTION

The present invention relates to cytokine receptor interactive molecules and their use in the development of new therapeutic and diagnostic molecules. More particularly, the present invention provides immunoreactive molecules such as antibodies capable of interacting with granulocyte colony stimulating factor receptor.

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

### BACKGROUND OF THE INVENTION

Granulocyte colony-stimulating factor (G-CSF) stimulates the proliferation and differentiation of neutrophil precursors via interaction with a specific cell surface receptor, the G-CSF receptor (G-CSF-R).

The G-CSF-R has been cloned (1) and is functionally active in several different cell types (2). However, little is known about the mechanism of signal transduction. The G-CSF-R is believed to consist of a single chain that is activated through ligand induced homodimerisation (3) as has been shown for the erythropoietin and growth hormone receptors (EPO-R, GH-R) (4). The G-CSF-R does not contain an intrinsic protein kinase domain (1) although tyrosine kinase activity seems to be required for transduction of the G-CSF signal (5).

In work leading up to the present invention, the inventors used immunological techniques to study the interaction between G-CSF and its receptor, G-CSF-R. The present invention provides for the first time immune reactive molecules capable of interacting with G-CSF-R which will facilitate the development of new therapeutic and diagnostic molecules based on the immune reactive molecules or agonists or antagonists thereof.

### SUMMARY OF THE INVENTION

One aspect of the present invention contemplates an immunointeractive molecule capable of binding or otherwise associating with an animal G-CSF receptor (G-CSF-R) extracellular domain. Preferably, the animal is a mammal such as a human or murine species. Preferably, the immunointeractive molecule is a polyclonal or monoclonal antibody.

Another aspect of the present invention is directed to a diagnostic agent comprising an immunointeractive molecule capable of binding or otherwise associating with an animal G-CSF-R extracellular domain. Preferably, the immunointeractive molecule is an antibody labelled with a reporter molecule.

Yet another aspect of the present invention relates to a pharmaceutical composition comprising an immunointeractive molecule as contemplated above together with one or more pharmaceutically acceptable carriers and/or diluents.

Still another aspect of the present invention provides a method for treating a G-CSF associated disease condition such as a cancer or tumour in a mammal, said method comprising administering to said mammal an effective amount of an immunointeractive molecule capable of binding or otherwise associating with an animal G-CSF-R extracellular domain.

### BRIEF DESCRIPTION OF THE FIGURES

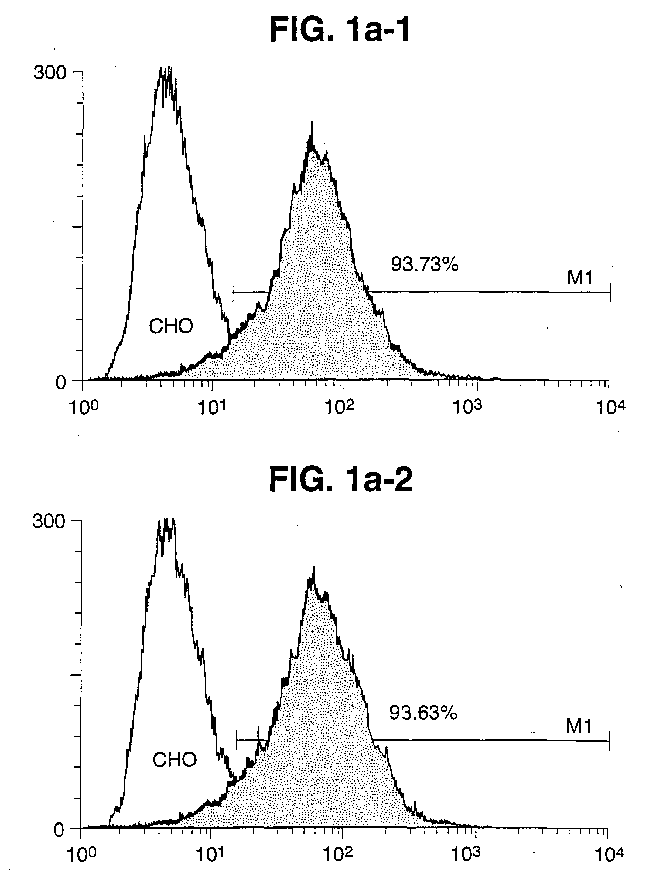

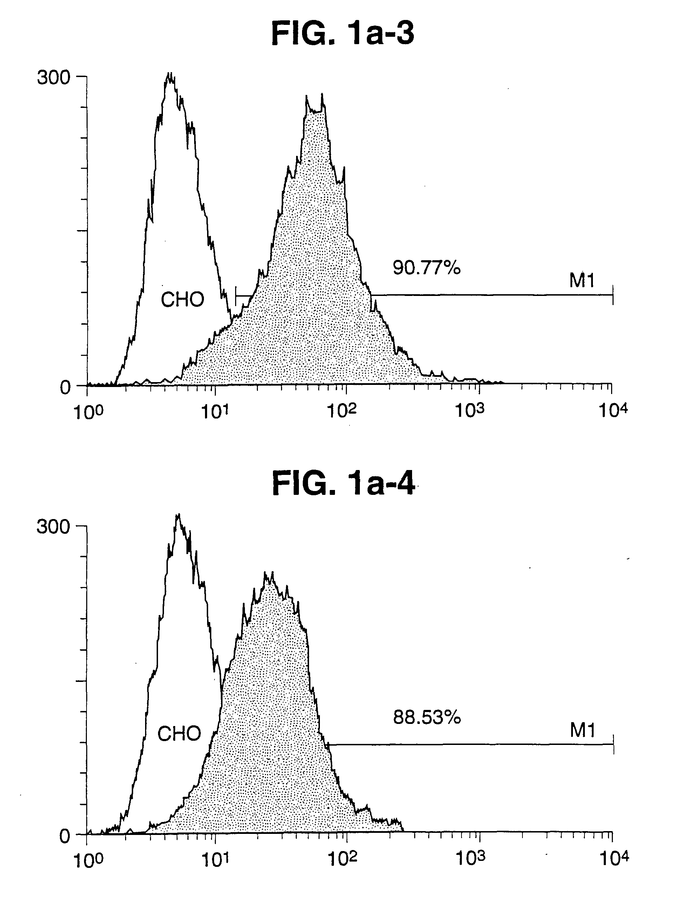

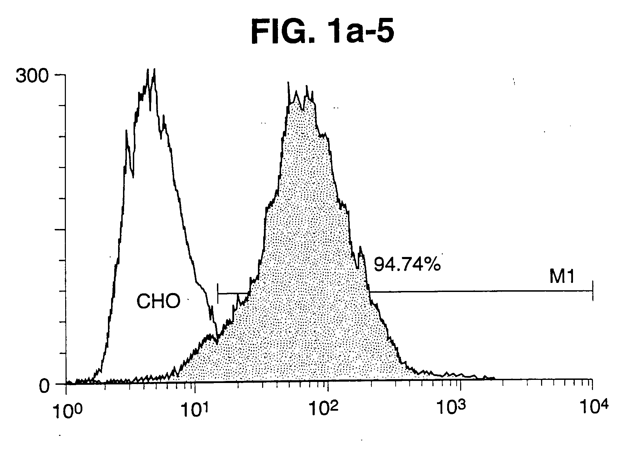

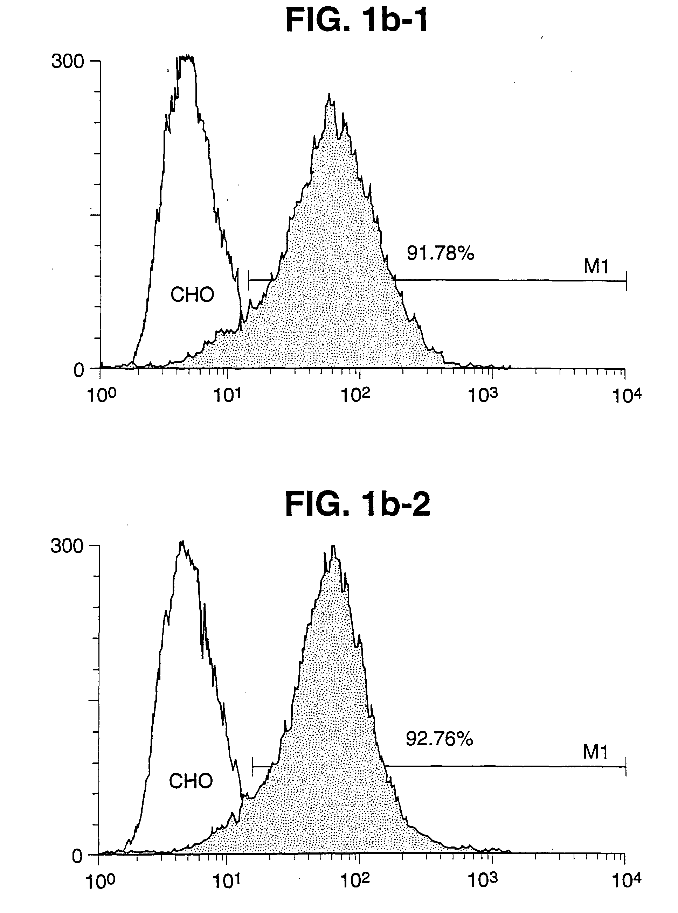

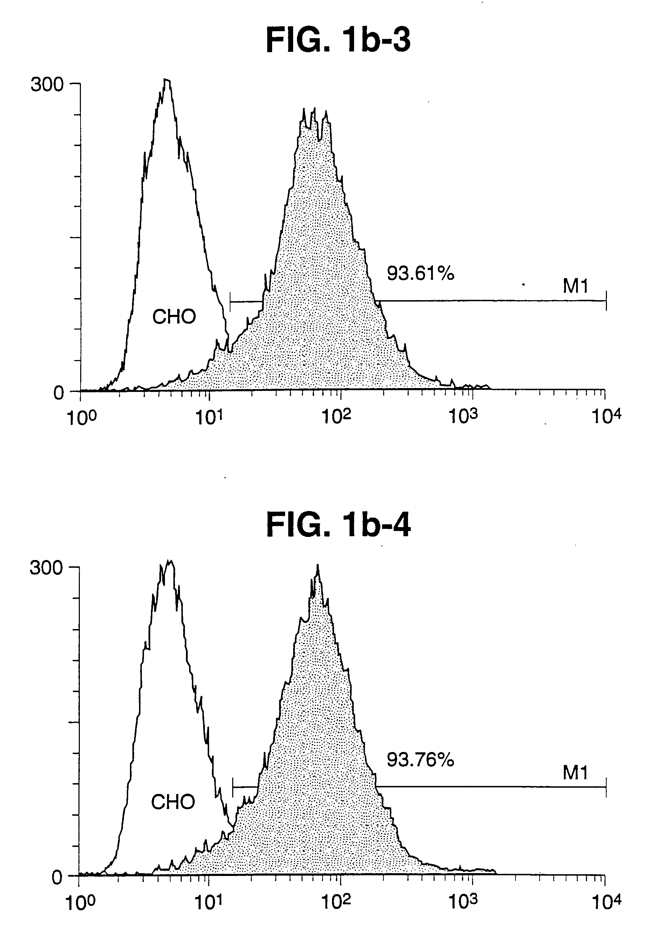

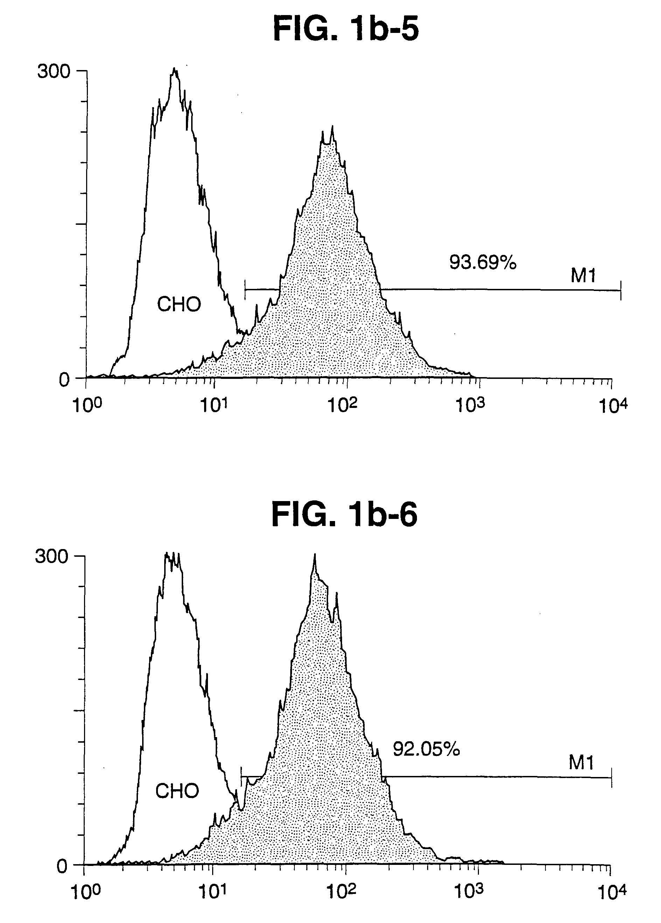

**Figure 1** is a FACS analysis of anti-G-CSF-R antibody binding to CHO-6A11 cells (solid histogram) compared with control CHO-10 cells (open histograms). The graphs show relative cell number versus fluorescence intensity (log scale).

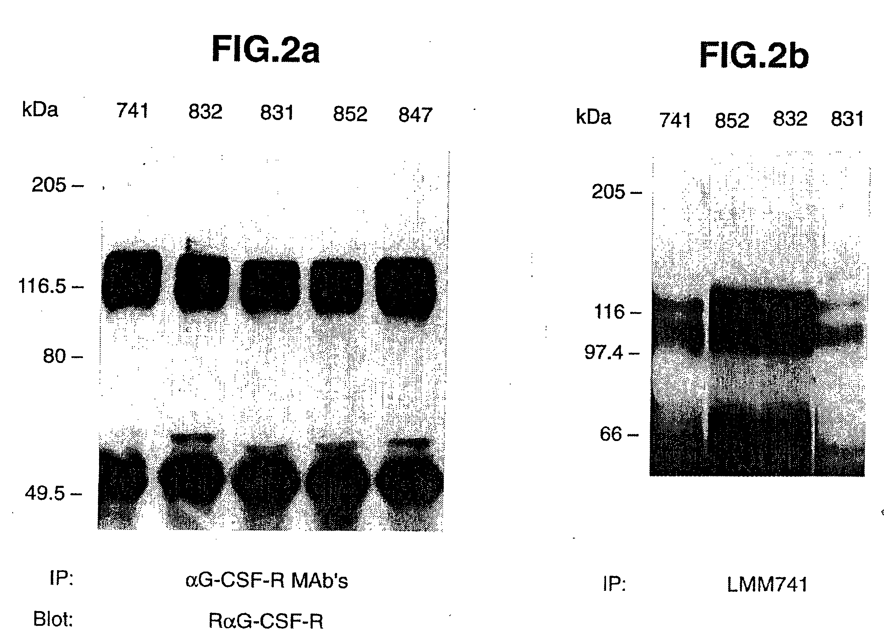

**Figure 2** shows immunoprecipitation and Western blotting assay of some of the anti-G-CSF-R antibodies. Left panel: CHO-6A11 cell lysates were immunoprecipitated with the test monoclonals, the precipitates run on SDS-PAGE and blotted with RαG-CSF-R antibody. Right panel: CHO-6A11 cell lysates were immunoprecipitated with LMM741, the precipitates run on SDS-PAGE and blotted with the test monoclonals. Results obtained with the antibodies not shown are given in Table 2.

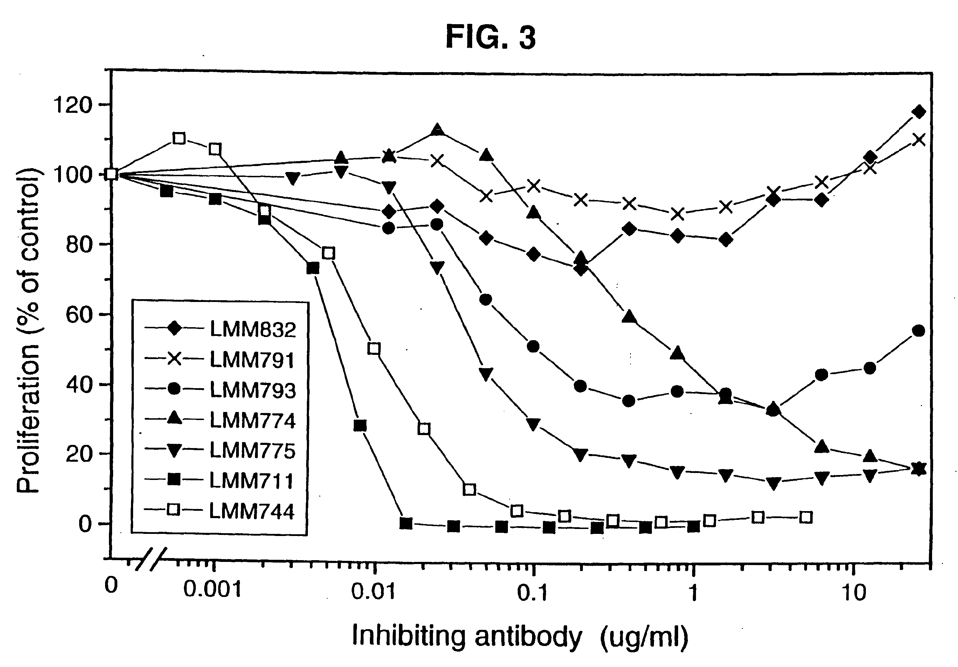

**Figure 3** shows inhibition of FD.hGR3 proliferation by anti-G-CSF-R antibodies. FD.hGR3 cells were cultured with G-CSF and antibody dilutions as described in the examples. The antibodies not shown did not cause inhibition, giving results that were similar to LMM791.

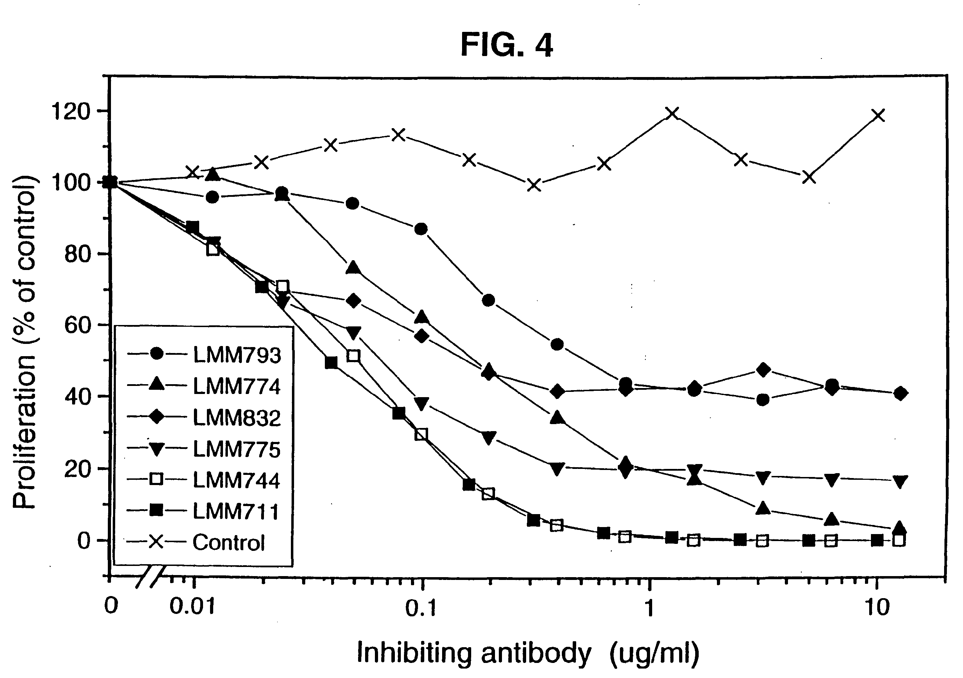

**Figure 4** shows inhibition of BAF.hGR proliferation by anti-G-CSF-R antibodies. BAF.hGR cells were cultured with G-CSF and antibody dilutions as described in the examples. Data from the antibodies not shown are given in Table 2.

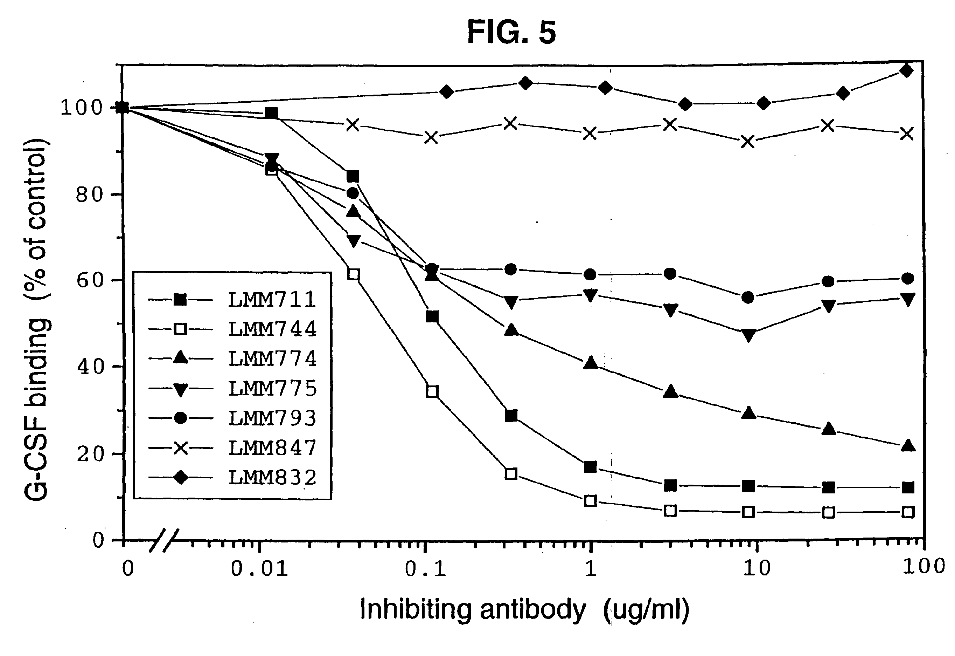

**Figure 5** shows inhibition of binding of ¹²⁵I-G-CSF to BAF.hGR cells by anti-G-CSF-R antibodies. The antibodies not shown gave results that were similar to LMM847.

### DETAILED DESCRIPTION OF THE PREFERRED EMBODIMENTS

One aspect of the present invention provides an immunointeractive molecule capable of binding or otherwise associating with an animal G-CSF-R, and in particular, the extracellular domain of such a receptor.

Preferably, the immunointeractive molecules are in the form of antibodies such as polyclonal or monoclonal antibodies although monoclonal antibodies are preferred. The present invention also extends to immunologically interactive fragments, parts, derivatives, homologues or analogues of these antibodies. Such antibodies may be in isolated or purified form meaning that a composition comprises at least 25 %, more preferably at least 35%, even more preferably at least 45-50%, still more preferably at least 60-75 % and even still more preferably at least 80-95 % of the antibodies as determined by weight, immunoreactivity or other convenient means. Alternatively, the antibodies may be present in the form of isolated culture supernatant, tissue extract, serum or whole blood or ascites fluid.

Preferably, the animal G-CSF-R is of mammalian origin such as from a human, livestock animal (e.g. cow, horse, sheep, goat or donkey), laboratory test animal (e.g. rat, mouse or rabbit), companion animal (e.g. dog or cat) or captive wild animal (e.g. dingo, fox, wild boar or kangaroo). The most preferred receptors are of human and laboratory test animal origin (e.g. murine species, i.e. rat or mouse).

Where the antibodies are polyclonal antibodies, they may be generated in any convenient host including a human, livestock animal, companion animal or captive wild animal as hereinbefore described. Where the antibodies are monoclonal antibodies, they may be prepared in any convenient hybridoma such as of murine (e.g. rat or mouse) origin.

The receptor used to generate the antibodies may be the whole receptor such as in purified, partially purified or isolated form including in the form of isolated membrane preparations. The receptor may also be produced by recombinant procedures or synthetic procedures or a combination thereof. In a particularly preferred embodiment, a fragment of the receptor is used which, in an even more preferred embodiment, is fused to a suitable carrier or marker molecule such as FLAG protein. Alternative carrier molecules include glutathione-S-transferase (GST) or alkaline phosphatase (AP).

According to this preferred embodiment, there is provided a molecule interactive with a non-full length G-CSF-R fused to a carrier molecule. The non-full length portion of the receptor acts as a hapten. Preferably, the non-full length receptor comprises its extracellular domain. Preferably, the carrier molecule is FLAG, AP or GST.

The resulting fusion molecule is then used to generate polyclonal or monoclonal antibodies which may undergo immunoadsorbent procedures to provide a composition of substantially, for example, extracellular domain-reactive receptor antibodies.

The term "immunointeractive molecules" is used herein in its broadest sense and includes antibodies, parts, fragments, derivatives, homologues or analogues thereof, peptide or non-peptide equivalents thereof and fusion molecules between two or more antibodies or between an antibody and another molecule. The antibodies or other immunointeractive molecules may also be in recombinant or synthetic form.

Accordingly, the present invention contemplates mutants and derivatives of the immunointeractive molecules, especially when such molecules are antibodies. Mutants and derivatives of such antibodies include amino acid substitutions, deletions and/or additions. Furthermore, amino acids may be replaced by other amino acids having like properties, such as hydrophobicity, hydrophilicity, electronegativity, bulky side chains, interactive and/or functional groups and so on. Glycosylation variants and hybrid antibodies are also contemplated by the present invention.

Amino acid substitutions are typically of single residues; insertions will usually be of the order of about 1-10 amino acid residues; and deletions will range from about 1-20 residues. Deletions or insertions preferably are made in adjacent pairs, i.e: a deletion of 2 residues or insertion of 2 residues.

The amino acid variants referred to above may readily be made using peptide synthetic techniques well known in the art, such as solid phase peptide synthesis and the like, or by recombinant DNA manipulations. Techniques for making substitution mutations at predetermined sites in DNA having known sequence are well known, for example through M13 mutagenesis. The manipulation of DNA sequences to produce variant proteins which manifest as substitutional, insertional or deletional variants are well known in the art.

Other examples of recombinant or synthetic mutants and derivatives of the antibodies of the present invention include single or multiple substitutions, deletions and/or additions to any molecule associated with the ligand such as carbohydrates, lipids and/or proteins or polypeptides. Naturally occurring or altered glycosylated forms of the subject antibodies are particularly contemplated by the present invention.

Amino acid alterations to the subject polypeptide contemplated herein include insertions such as amino acid and/or carboxyl terminal fusions as well as intrasequence insertions of single or multiple amino acids. Generally, insertions within the amino acid sequence will be smaller than amino or carboxyl terminal fusions, of the order of about 1 to 4 residues. Insertional amino acid sequence variants are those in which one or more amino acid residues are introduced into a predetermined site in the protein. Deletional variants are characterised by the removal of one or more amino acids from the sequence. Substitutional variants are those in which at least one residue in the sequence has been removed and a different residue inserted in its place. Such substitutions may be made in accordance with Table 1.

**TABLE 1**

| Original Residue | Exemplary Substitutions |
|---|---|
| Ala | Ser |
| Arg | Lys |
| Asn | Gln; His |
| Asp | Glu |
| Cys | Ser |
| Gln | Asn |
| Glu | Asp |
| Gly | Pro; Val |
| His | Asn; Gln |
| Ile | Leu; Val |
| Leu | Ile; Val |
| Lys | Arg; Gln; Glu |
| Met | Leu; Ile |
| Phe | Met; Leu; Tyr |
| Ser | Thr |
| Thr | Ser |
| Trp | Tyr |
| Tyr | Trp; Phe |
| Val | Ile; Leu |

The terms "analogues" and "derivatives" also extend to any functional chemical equivalents of the antibodies characterised by their increased stability and/or efficacy *in vivo* or *in vitro.* The terms "analogue" and "derivatives" further extend to any amino acid derivative of the antibodies as described above.

Antibody analogues contemplated herein include, but are not limited to, modifications to side chains, incorporation of unnatural amino acids and/or derivatising the molecules and the use of crosslinkers and other methods which impose conformational constraints on the antibodies. Examples of side chain modifications contemplated by the present invention include modifications of amino groups such as by reductive alkylation by reaction with an aldehyde followed by reduction with NaBH₄; amidination with methylacetimidate; acylation with acetic anhydride; carbamoylation of amino groups with cyanate; trinitrobenzylation of amino groups with 2, 4, 6-trinitrobenzene sulphonic acid (TNBS); acylation of amino groups with succinic anhydride and tetrahydrophthalic anhydride; and pyridoxylation of lysine with pyridoxal-5'-phosphate followed by reduction with NaBH₄.

The guanidine group of arginine residues may be modified by the formation of heterocyclic condensation products with reagents such as 2,3- butanedione, phenylglyoxal and glyoxal.

The carboxyl group may be modified by carbodiimide activation *via* O-acylisourea formation followed by subsequent derivitisation, for example, to a corresponding amide.

Sulphydryl groups may be modified by methods such as carboxymethylation with iodoacetic acid or iodoacetamide; performic acid oxidation to cysteic acid; formation of a mixed disulphides with other thiol compounds; reaction with maleimide, maleic anhydride or other substituted maleimide; formation of mercurial derivatives using 4-chloromercuribenzoate, 4-chloromercuriphenylsulphonic acid, phenylmercury chloride, 2-chloromercuri-4-nitrophenol and other mercurials; carbomoylation with cyanate at alkaline pH.

Tryptophan residues may be modified by, for example, oxidation with N-bromosuccinimide or alkylation of the indole ring with 2-hydroxy-5-nitrobenzyl bromide or sulphenyl halides. Tyrosine residues on the other hand, may be altered by nitration with tetranitromethane to form a 3-nitrotyrosine derivative.

Modification of the imidazole ring of a histidine residue may be accomplished by alkylation with iodoacetic acid derivatives or N-carbethoxylation with diethylpyrocarbonate.

Examples of incorporating unnatural amino acids and derivatives during protein synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids.

Crosslinkers can be used, for example, to stabilise 3D conformations, using homo-bifunctional crosslinkers such as the bifunctional imido esters having (CH₂)ₙ spacer groups with n = 1 to n = 6, glutaraldehyde, N-hydroxysuccinimide esters and hetero-bifunctional reagents which usually contain an amino-reactive moiety such as N-hydroxysuccinimide and another group specific-reactive moiety such as maleimido or dithio moiety (SH) or carbodiimide (COOH). In addition, peptides could be conformationally constrained by, for example, incorporation of C_{α} and N_{α}-methylamino acids, introduction of double bonds between C_{α} and C_{β} atoms of amino acids and the formation of cyclic peptides or analogues by introducing covalent bonds such as forming an amide bond between the N and C termini, between two side chains or between a side chain and the N or C terminus.

The present invention, therefore, extends to amino acid and/or chemical analogues of the subject antibodies having the identifying characteristics of being interactive with the extracellular domain of the NYK-flk-1 receptor.

Accordingly, reference herein to an antibody includes the naturally occurring molecule, recombinant, synthetic and analogue forms thereof and to any mutants, derivatives and human and non-human homologues thereof including amino acid and glycosylation variants.

The immunointeractive molecules of the present invention may be used to develop a new range of therapeutic and diagnostic agents. For example, the antibodies or fragments or derivatives thereof may act as antagonists to disrupt G-CSF and receptor interaction useful which may be useful in the treatment of some tumours and cancers. They may also be used for screening for agonists useful, for example, where G-CSF interaction with its receptor is to be promoted. Normal, abnormal or mutated receptor structure or receptor expression can also be determined through immunoreactivity studies.

According to this latter embodiment, there is contemplated a method of detecting a G-CSF-R on a cell in a biological sample, said method comprising contacting said sample with an immunointeractive molecule capable of binding to the extracellular domain of said NYK/flk-1 receptor immobilised to a solid support for a time and under conditions sufficient for an immunointeractive molecule-G-CSF-R complex to form and then detecting the presence of said complex.

In one preferred method, the immunointeractive molecule in G-CSF-R complex is detected by contacting the complex with an antibody against the immunointeractive molecule with the antibody being labelled with a reporter molecule. Alternatively, the immunointeractive molecule itself is labelled with a reporter molecule.

The immunointeractive molecules are generally antibodies and these or antibodies directed against the immunointeractive molecules may be polyclonal or monoclonal antibodies and these are obtainable by immunisation of a suitable animal with the immunointeractive molecule (or G-CSF-R) and either type is utilisable in the assay. The methods of obtaining both types of sera are well known in the art. Polyclonal sera are less preferred but are relatively easily prepared by injection of a suitable laboratory animal with an effective amount of immunointeractive molecule or G-CSF-R preparation, or antigenic parts thereof, collecting serum from the animal, and isolating specific antibodies by any of the known immunoadsorbent techniques. Although antibodies produced by this method are utilisable in virtually any type of assay, they are generally less favoured because of the potential heterogeneity of the product.

The use of monoclonal antibodies in the above immunoassay is particularly preferred because of the ability to produce them in large quantities and the homogeneity of the product. The preparation of hybridoma cell lines for monoclonal antibody production derived by fusing an immortal cell line and lymphocytes sensitised against the immunogenic preparation can be done by techniques which are well known to those who are skilled in the art. (See, for example Douillard and Hoffman, Basic Facts about Hybridomas, in *Compendium of Immunology* Vol II, ed. by Schwartz, 1981; Kohler and Milstein, *Nature 256:* 495-499, 1975; *European Journal of Immunology 6:* 511-519, 1976).

The presence of a G-CSF-R receptor may be accomplished in a number of ways such as by Western blotting and ELISA procedures. A wide range of immunoassay techniques are available as can be seen by reference to US Patent Nos. 4,016,043, 4, 424,279 and 4,018,653. These, of course, include both single-site and two-site or "sandwich" assays of the non-competitive types, as well as in the traditional competitive binding assays. These assays also include direct binding of a labelled antibody to a target.

Sandwich assays are among the most useful and commonly used assays and are particularly useful in the present invention. A number of variations of the sandwich assay technique exist, and all are intended to be encompassed by the present invention. Briefly, in a typical forward assay, an immunointeractive molecule is brought into contact with a biological sample comprising cells potentially carrying G-CSF-R. After a suitable period of incubation, for a period of time sufficient to allow formation of an immunointeractive molecule-G-CSF-R complex, an antibody specific to the immunointeractive molecule, labelled with a reporter molecule capable of producing a detectable signal, is then added and incubated allowing sufficient time for the formation of a tertiary complex. Any unreacted material is washed away, and the presence of the antibody bound to the immunointeractive molecule is determined by observation of a signal produced by the reporter molecule. The results may either be qualitative, by simple observation of the visible signal, or may be quantitated by comparing with a control sample containing known amounts of hapten. Variations on the forward assay include using an immunointeractive molecule labelled with a reporter molecule. In addition, the immunointeractive molecule or cells may be immobilised onto a solid support.

Suitable solid supports include glass or a polymer, the most commonly used polymers being cellulose, polyacrylamide, nylon, polystyrene, polyvinyl chloride or polypropylene. The solid supports may .be in the form of tubes, beads, discs of microplates, or any other surface suitable for conducting an immunoassay. The binding processes are well-known in the art and generally consist of cross-linking covalently binding or physically adsorbing the molecules to the polymer.

"Reporter molecule", as used in the present specification, is meant a molecule which, by its chemical nature, provides an analytically identifiable signal which allows the detection of antigen-bound antibody. Detection may be either qualitative or quantitative. The most commonly used reporter molecules in this type of assay are either enzymes, fluorophores or radionuclide containing molecules (i.e. radioisotopes) and chemiluminescent molecules.

In the case of an enzyme immunoassay, an enzyme is conjugated to the immunointeractive molecule or an antibody thereto generally by means of glutaraldehyde or periodate. As will be readily recognised, however, a wide variety of different conjugation techniques exist, which are readily available to the skilled artisan. Commonly used enzymes include horseradish peroxidase, glucose oxidase, beta-galactosidase and alkaline phosphatase, amongst others. The substrates to be used with the specific enzymes are generally chosen for the production, upon hydrolysis by the corresponding enzyme, of a detectable colour change. Examples of suitable enzymes include alkaline phosphatase and peroxidase. It is also possible to employ fluorogenic substrates which yield a fluorescent product rather than the chromogenic substrates noted above. Generally, the enzyme-labelled antibody is added to the immunointeractive molecule-receptor complex, allowed to bind, and then the excess reagent is washed away. Alternatively, an enzyme labelled immunointeractive molecule is used. A solution containing the appropriate substrate is then added to the tertiary complex. The substrate will react with the enzyme linked to the antibody/immunointeractive molecule, giving a qualitative visual signal, which may be further quantitated, usually spectrophotometrically, to give an indication of the amount of hapten which was present in the sample. "Reporter molecule" also extends to use of cell agglutination or inhibition of agglutination such as red blood cells on latex beads, and the like.

Alternately, fluorescent compounds, such as fluorescein and rhodamine, may be chemically coupled to antibodies without altering their binding capacity. When activated by illumination with light of a particular wavelength, the fluorochromelabelled antibody adsorbs the light energy, inducing a state to excitability in the molecule, followed by emission of the light at a characteristic colour visually detectable with a light microscope. Immunofluoresence and EIA techniques are both very well established in the art and are particularly useful for the present method. However, other reporter molecules, such as radioisotope, chemiluminescent or bioluminescent molecules, may also be employed.

By detecting G-CSF-R by the procedures, aberrant receptors may be discerned thus providing a useful screening procedure for potential disease conditions.

Furthermore, the present invention also provides a method of screening for G-CSF-R, such as an altered G-CSF-R, by determining the ability of a putative G-CSF-R to interact with an antibody thereto. The method may also be employed to scren for molecules capable of disrupting G-CSF-R antibody interaction thereby providing potential antagonists. The method may also be used to screen for enhanced antibody binding thereby providing potential agaonists.

The present invention also provides a pharmaceutical composition comprising an effective amount of an immunointeractive molecule capable of binding or otherwise associating with the extracellular domain of G-CSF-R and one or more pharmaceutically acceptable carriers and/or diluents. The active ingredients of a pharmaceutical composition comprising the immunointeractive molecules are contemplated to exhibit excellent therapeutic activity, for example, in the treatment of angiogenic-dependent phenotype and disease conditions resulting therefrom, such as metastasis, in an amount which depends on the particular case. For example, from about 0.5 *µ*g to about 20 mg per kilogram of body weight per day may be administered. Dosage regima may be adjusted to provide the optimum therapeutic response. For example, several divided doses may be administered daily or the dose may be proportionally reduced as indicated by the exigencies of the therapeutic situation.

The active compound may be administered in a convenient manner such as by the oral, intravenous (where water soluble), intramuscular, subcutaneous, intranasal, intradermal or suppository routes or implanting (eg using slow release molecules). Depending on the route of administration, the active ingredients which comprise the immunointeractive molecules may be required to be coated in a material to protect said ingredients from the action of enzymes, acids and other natural conditions which may inactivate said ingredients. In order to administer the immunointeractive molecules by other than parenteral administration, they will be coated by, or administered with, a material to prevent its inactivation. For example, the immunointeractive molecules may be administered in an adjuvant, co-administered with enzyme inhibitors or in liposomes. Adjuvant is used in its broadest sense and includes any immune stimulating compound such as interferon. Adjuvants contemplated herein include resorcinols, non-ionic surfactants such as polyoxyethylene oleyl ether and n-hexadecyl polyethylene ether. Enzyme inhibitors include pancreatic trypsin inhibitor, diisopropylfluorophosphate (DEP) and trasylol. Liposomes include water-in-oil-in-water emulsions as well as conventional liposomes.

The active compounds may also be administered parenterally or intraperitoneally. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions (where water soluble) or dispersions and sterile powders for the extemporaneous preparation of sterile injectable solutions or dispersion. In all cases the form must be sterile and must be fluid to the extent that easy syringability exists. It must be stable under me conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as licithin, by the maintenance of the required particle size in the case of dispersion and by the use of superfactants. The prevention of the action of microorganisms can be brought about by various antibacterial and antifungal agents, for example, parabens, chlorobutanol, phenol, sorbic acid, thirmerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

When immunointeractive molecules are suitably protected as described above, the active, compound may be orally administered, for example, with an inert diluent or with an assimilable edible carrier, or it may be enclosed in hard or soft shell gelatin capsule, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups, wafers, and the like. Such compositions and preparations should contain at least 1% by weight of active compound. The percentage of the compositions and preparations may, of course, be varied and may conveniently be between about 5 to about 80% of the weight of the unit. The amount of active compound in such therapeutically useful compositions in such that a suitable dosage will be obtained. Preferred compositions or preparations according to the present invention are prepared so that an oral dosage unit form contains between about 10 *µ*g and 2000 mg of active compound.

The tablets, troches, pills, capsules and the like may also contain the following: A binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such a sucrose, lactose or saccharin may be added or a flavouring agent such as peppermint, oil of wintergreen, or cherry flavouring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavouring such as cherry or orange flavour. Of course, any material used in preparing any dosage unit form should be pharmaceutically pure and substantially non-toxic in the amounts employed. In addition, the active compound may be incorporated into sustained-release preparations and formulations.

As used herein "pharmaceutically acceptable carrier and/or diluent" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, use thereof in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in dosage unit form as hereinbefore disclosed. A unit dosage form can, for example, contain the principal active compound in amounts ranging from 0.5 *µ*g to about 2000 mg. Expressed in proportions, the active compound is generally present in from about 0.5 *µ*g to about 2000 mg/ml of carrier. In the case of compositions containing supplementary active ingredients, the dosages are determined by reference to the usual dose and manner of administration of the said ingredients.

In a most preferred embodiment, the immunointeractive molecules used in a pharmaceutical composition are antibodies or mutants or derivatives thereof. Most preferably, the antibodies are monoclonal antibodies.

The present invention is further described by reference to the following nonlimiting examples.

### EXAMPLE 1

### CHO-6A11 CELLS

CHO-K1 cells were transfected by the calcium phosphate method (6) with the pEE6.HCMV.GS expression vector (7,8) carrying a full length cDNA encoding the human G-CSF-R (9). Transfected cells were selected for glutamine synthetase expression by growth in GMEM-S medium (10). Colonies were further tested for expression of the G-CSF-R by determining their ability to bind ¹²⁵I-G-CSF. The colony with the highest level of G-CSF binding (CHO-6A11) was selected for further use.

### EXAMPLE 2

### FACTOR DEPENDENT CELLS

FDC-P1 cells and BAF/BO3 cells were co-transfected by electroporation with the pEFBos expression vector (11) carrying the G-CSF-R cDNA and pGKneo (12). Transfected cells were selected by growth in medium containing 1.2 mg/ml neomycin. Growing colonies were expanded and tested for proliferative response to G-CSF. Positive colonies were cloned and adapted to growth in G-CSF instead of WEHI-3 D⁻ cell conditioned medium (a source of GM-CSF and IL-3), resulting in clone FD-hGR3 and several BAF.hGR clones, which could be grown long term in G-CSF as the only growth factor.

### EXAMPLE 3

### RABBIT ANTI-G-CSF-R ANTISERUM

Rabbits were immunised with a glutathione-S-transferase-G-CSF-R fusion protein (GST-GR) containing the haemopoietin receptor domain. A BamH1 fragment of the receptor cDNA encoding amino acids 17-345 was sub-cloned into the BamH1 site of pGEX-1 (13) and the construct was expressed in *E*. *coli.* The fusion protein was partially purified by affinity chromatography as described (13). Rabbits were injected with 100 *µ*g GST-GR emulsified in complete Freund's adjuvant subcutaneously. After 6 weeks, rabbits were boosted with 100 *µ*g GST-GR in incomplete Freund's adjuvant then bled after a further 2 weeks. The best antiserum was designated R55.

### EXAMPLE 4

### HYBRIDOMA PRODUCTION

BALB/c mice were immunised with about 2 x 10⁷ CHO-6A11 cells intraperitoneally in PBS. The mice were boosted after 2 and 4 weeks with the same inoculum. At least one month after the last boost, mice were given a final boost I/P and their spleens were fused 4 days later with P3-NS1/1-Ag4-11 murine myeloma cells by standard methods (14).

Two approaches were used to screen hybrids for production of anti-receptor antibody. The early fusions were first screened for production of IgG antibody by an ELISA and positive wells were then screened for binding to CHO-6A11 cells, but not to CHO cells transfected with an irrelevant receptor (CHO-10 cells) by fluorescence microscopy. After several antibodies had been obtained with this procedure, an ELISA was established with glutaraldehyde-fixed CHO-6A11 and control CHO cells. 5 x 10⁴ cells/well were aliquotted into flat bottomed 96-well plates in 50 *µ*l PBS and centrifuged. Glutaraldehyde (0.05 % v/v, 200 *µ*l/well) was added and the cells allowed to fix for 10 min at room temperature. Plates were then washed with PBS containing 0.02% v/v Tween 20 four times and stored in PBS at 4°C until required. Using 0.05% w/v glutaraldehyde allowed all the antibodies tested to recognise the CHO-6A11 cells; higher concentrations destroyed some epitopes. Antibody binding was detected with biotinylated anti-γ chain antibody (Amersham) followed by avidin-biotinylated peroxidase complex (Dako). Antibodies that were positive in this assay were also tested by fluorescence.

### EXAMPLE 5

### ANTIBODY PURIFICATION

Hybridoma culture supernatant was precipitated with 50% V/V saturated ammonium sulphate at 4°C and the precipitate was dissolved in deionised water then dialysed against PBS. IgG antibodies were further purified by Protein A-Sepharose affinity chromatography.

### EXAMPLE 6

### FLUORESCENCE ANALYSIS

CHO-6A11 and CHO-10 cells were harvested using PBS containing 2 MM EDTA and resuspended in PBS containing 10% v/v fetal calf serum (PBS-FCS). Monoclonal antibodies were added at a final concentration of 10 *µ*g/ml and incubated for 30 min at 4°C. Cells were washed and resuspended in FITC-conjugated sheep anti-mouse Ig (Silenus) diluted 1:100 in PBS-FCS. Samples were incubated for 30 min at 4°C then washed twice. Cells were resuspended in PBS-FCS and propidium iodide was added to a final concentration of 2 *µ* g/ml to stain dead cells. Cells were analysed in a FACScan with Lysis II software (Becton Dickinson).

### EXAMPLE 7

### IMMUNOPRECIPITATION AND WESTERN BLOTTING

CHO-6A11 cells were lysed in lysis buffer (50 mM Tris/HCl, 150 mM NaCl, 1 % v/v Triton-X-100, 1 mM EDTA and protease inhibitors) and the protein concentration of the lysate was determined by Bradford assay (Biorad) using bovine serum albumin as a standard. Aliquots of cell lysate were pre-cleared with rabbit anti-mouse immunoglobulin (Dakopatts) and protein A-Sepharose (Pharmacia) for 2 hr at 4°C. The cleared lysates were incubated with monoclonal antibodies (3 *µ*g/ml purified antibody or 20 *µ*l/ml of hybridoma supernatant) for 2 hr at 4°C, then complexes were precipitated with rabbit anti-mouse immunoglobulin and protein A-Sepharose. Immunoprecipitates were analysed by SDS-polyacrylamide electrophoresis (PAGE), reducing conditions, on a 6% w/v acrylamide gel. Protein was then electrophoretically transferred to nitrocellulose and blocked overnight at 4°C with 10% w/v skim milk powder solution. After washing in PBS/0.1% v/v Tween 20, the nitrocellulose was incubated with rabbit anti-G-CSF-R antibody purified on protein A-Sepharose (R55, 0.4 *µ*g/ml) followed by peroxidaseconjugated swine anti-rabbit immunoglobulin (1:1000, Dakopatts) and enhanced chemiluminescence (ECL) (Amersham).

To test the ability of the monoclonal antibodies to detect the G-CSF-R by Western blotting, the receptor was immunoprecipitated with 15 *µ*l of a 50% slurry of Sepharose-conjugated LMM741 (5 mg antibody / ml Sepharose) and blotted with monoclonal anti-receptor antibodies (0.5 *µ*g/ml) followed by peroxidase-conjugated rabbit anti-mouse immunoglobulin (1:1000, Dakopatts) and ECL.

### EXAMPLE 8

### PROLIFERATION ASSAYS

FD.hGR3 cells or BAG.hGR cells were washed 3 times and resuspended in DME medium with 10% v/v heat inactivated fetal calf serum at 5 x 10⁵/ml (FD.hGR) or 1 x 10⁵/ml (BAF.hGR). Monoclonal antibodies were diluted to 400 *µ*g/ml in medium and usually 2-fold serial dilutions were performed. Recombinant hG-CSF (Amgen) was diluted to 2 ng/ml in medium. Fifty *µ*l of antibody dilution, 50 *µ*l of G-CSF and 100 *µ*l of cell suspension were added to wells of 96-well tissue culture plates (Nunc) in triplicates. Plates were incubated for 24 hr at 37°C. After 20 hr or 44 hr, respectively, of incubation, plates were pulsed with 0.5 *µ*Ci/well ³H-thymidine for 4 hr then harvested onto glass fibre filter paper and counted in an LKB Betaplate liquid scintillation counter.

### EXAMPLE 9

### G-CSF BINDING ASSAY

YPY-rh-G-CSF (Tyr 1,3-G-CSF) (Kirin) was iodinated by the lactoperoxidase-glucose oxidase method (15), giving a specific activity of 0.9-3 x 10⁸ cpm/*µ*g, as determined by self displacement analysis (16) or ELISA. BAF.hGR cells were cultured overnight in medium containing WEHI-3B D⁻ conditioned medium instead of G-CSF before use. Antibodies to be tested wre diluted in RPMI medium containing 10 mM HEPES buffer and 10% v/v FCS (RHF). The cells were washed once with RPMI, resuspended in cold RHF and diluted to 3.3 x 10⁶/ml. Antibody dilutions were added (50 *µ*l/tube) to 1.5 ml tubes followed by 100 *µ*l of RHF or unlabelled rhG-CSF (3*µ*g/ml, 50*µ*l of ¹²⁵I-G-CSF (approximately 40,000 cpm) and 300 *µ*l of cell suspension. Tubes were incubated for 4 hr at 4°C on a rotator to give equilibrium binding. Cells were spun down, washed through 0.2 ml FCS in a 0.4 ml tube and the bottom of the tube containing the cell pellet was cut off and counted in a γ-counter (Packard).

Table 2 below, is a summary of the characteristics of the anti-G-CSF-R antibodies. The dashes indicate that the antibody had no effect. The proliferation and binding data are given as the concentration of antibody giving 50% inhibition, or where there was only partial inhibition, as percentage inhibition.

**TABLE 2**

| Anti-G-CSF-R Antibody Characteristics | | | | | | |
|---|---|---|---|---|---|---|
| Antibody | Isotype | FITC Mean Ch. | IP | Western | Bioassay (FD-GR3) | Binding Inhibition |
| LMM 711 | IgG1 *K* | 77 | + | - | +++ | ++ |
| LMM 744 | IgG2a *K* | 32 | - | - | ++ | ++ |
| LMM 772 | IgG1 *K* | 404 | +/- | - | + | + |
| LMM 774 | IgG1 *K* | 73 | + | - | + | ++ |
| LMM 775 | IgG1 *K* | 84 | + | - | ++ | + |
| LMM 793 | IgG1 *K* | 85 | + | +/- | + | + |
| LMM 791 | IgG1 *K* | 70 | + | + | - | - |
| LMM 741 | IgG1 *K* | 88 | + | + | - | - |
| LMM 831 | IgG2a *K* | 83 | + | + | - | - |
| LMM 832 | IgG1 *K* | 77 | + | + | - | - |
| LMM 847 | IgG2a *K* | 76 | + | + | - | - |
| LMM 852 | IgG1 *K* | 80 | + | + | - | - |
| LMM 792 | IgG1 *K* | 38 | - | +/- | - | - |
| LMM 776 | IgG1 *K* | 383 | - | - | - | - |
| LMM 777 | IgG1 *K* | 386 | - | - | - | - |
| LMM 825 | IgM *K* | 146 | - | - | - | - |

### REFERENCES:

1. R. Fukunaga, et al., Proc. Natl. Acad. Sci. USA 87: 8702 (1990); Larsen et al J. Exp. Med. 172: 1559 (1990); R. Fukunaga et al., Cell 61: 341 (1990).
2. R. Fukunaga, et al., EMBO 10: 2855 (1991).
3. R. Fukunaga et al., Proc. Natl. Acad. Sci. USA 90: 123 (1993).
4. S. Watawich et al., Proc. Natl. Acad. Sci. USA 89: 2140 (1992); G. Fuh et al., Science 256: 1677 (1992).
5. R.J. Isfort et al., Growth Factors 2: 213 (1990).
6. Gorman, C. (1985) in DNA Cloning: A Practical Approach, ed. D.M. Glover, IRL Press, Oxford, Vol. II ch. 6.
7. Stephens, P.E. et al., Nucleic Acids Res. 17: 7110 (1989).
8. Cockett, M.I., et al., Biotechnology 8: 662 (1990).
9. Fukunaga, R., et al., Proc. Natl. Acad. Sci. USA 87: 8702 (1990).
10. Bebbington, C.R. and Hentschel, C.C.G. (1985) in DNA Cloning: A Practical Apporach, ed. D.M. Glover, IRL Press, Oxford, Vol III Ch. 8.
11. Mizushima, S., et al., Nucleic Acids Res. 18: 5322 (1990).
12. Tybulewicx, V.L.J. et al., Cell 65: 1153 (1991).
13. Smith, D.B., et al., Gene 67: 31 (1988).
14. Brodsky, F.M., et al., Tissue Antigens 16: 30 (1980).
15. Hanazono, Y., et al., Exp. Hematol. 18: 1097 (1990).
16. Calvo, J.C., et al., Biochem. J. 212: 259 (1983).

PARAGCAPH 1(P1)An immunointeractive molecule capable of binding to or associating with an animal G-CSF receptor extracellular domain.
P2 The immunointeractive molecule of P1 wherein said animal is a mammal.
P3 The immunointeractive molecule of P2 wherein the mammal is a human, livestock animal, laboratory test animal, companion animal or captive wild animal.
P4 The immunointeractive molecule of P3 wherein the mammal is a human or a murine animal.
P5 The immunointeractive molecule of P1 wherein the molecule is an antibody.
P6 The immunointeractive molecule of P5 wherein the antibody is a polyclonal antibody.
P7 The immunointeractive molecule of P5 wherein the antibody is a monoclonal antibody.
P8 The immunointeractive molecule of P1 wherein the G-CSF receptor extracellular domain is a non-full length molecule fused to a carrier molecule.
P9 The immunointeractive molecule of P8 wherein the carrier molecule is FLAG protein.
P10 The immunointeractive molecule of P8 wherein the carrier molecule is glutathione-S-transferase.
P11 A diagnostic agent comprising an immunointeractive molecule capable of binding to or associating with an animal G-CSF receptor extracellular domain.
P12 The diagnostic agent of P11 wherein said animal is a mammal.
P13 A diagnostic agent of P12 wherein said mammal is a human or a murine animal.
P14 The diagnostic agent of P11 wherein the molecule is an antibody.
P15 The diagnostic agent of P14 wherein the antibody is a polyclonal antibody.
P16 The diagnostic agent of P14 wherein the antibody is a monoclonal antibody.
P17 The diagnostic agent of P14, P15 or P16 further comprising a reporter molecule fused to said antibody.
P18 A pharmaceutical composition comprising an immunointeractive molecule capable of binding to or associating with an animal G-CSF receptor extracellular domain, said composition further comprising one or more pharmaceutical carriers and/or diluents.
P19 The pharmaceutical composition of P18 wherein said animal is a mammal.
P20 The pharmaceutical composition P19 wherein the mammal is a human or a murine animal.
P21 The pharmaceutical composition of P18 wherein the molecule is an antibody.
P22 The pharmaceutical composition of P21 wherein the antibody is a polyclonal antibody.
P23 The pharmaceutical composition of P22 wherein the antibody is a monoclonal antibody.
P24 A method for inhibiting or decreasing G-CSF dependent cell responses or disease conditions resulting therefrom in a mammal comprising administering to said mammal an effective amount of an immunointeractive molecule capable of binding to or associating with an animal G-CSF receptor extracellular domain.
P25 The method of P24 wherein said mammal is a human.
P26 The method of P24 or P25 wherein the molecule is an antibody.
P27 The method of P26 wherein said antibody is a polyclonal antibody.
P28 The method of P26 wherein said antibody is a monoclonal antibody.
P29 The method of P24 wherein the disease condition is cancer.

## Claims

1. A method for the production of antibodies capable of binding to or associating with mammalian G-CSF receptor comprising immunising an animal with a cell-line expressing the extracellular domain of G-CSF receptor.

2. A method for the production of a monoclonal antibody comprising:
i) immunising an animal with a cell-line expressing the extracellular domain of the mammalian G-CSF-receptor;
ii) isolating a cell sample comprising antibody producing cells;
iii) fusing antibody producing cells with myeloma cells to produce a hybridoma cell-line; and
iv) cloning said hybridoma cell-line.

3. A method according to claim 1 or 2 wherein the cell-line is Chinese Hamster Ovary cells (CHO).

4. A method according to any of claims 1-3 wherein the G-CSF- receptor is human.

5. A hybridoma cell-line obtainable by the method according to any of claims 2-4.

6. Antibodies capable of antagonising the activity of G-CSFR obtainable by the method according to any of claims 1-4.

7. Antibodies capable of agonising the activity of G-CSFR obtainable by the method according to any of claims 1-4.

8. A pharmaceutical composition comprising an antibody according to claim 6 and a carrier or diluent.

9. A pharmaceutical composition comprising an antibody according to claim 7 and a carrier or diluent.

10. Use of an antibody according to Claim 6 for the manufacture of a medicament for use in the treatment of cancer.
